# EUROPEAN PATENT SPECIFICATION

(11) **EP 3 689 856 B1**
(45) Date of publication and mention of the grant of the patent: **02.06.2021**
(21) Application number: 19182458.0
(22) Date of filing: 26.06.2019
(51) Int. Cl.: C07D 285/08, C07D 285/12, H05B 33/00, C09K 11/06

(54) **SYMMETRICAL IMINES AND A METHOD OF SYNTHESIZING SYMMETRICAL IMINES**
SYMMETRISCHE IMINE UND VERFAHREN ZUR HERSTELLUNG VON SYMMETRISCHEN IMINEN
IMINES SYMÉTRIQUES ET PROCÉDÉ DE SYNTHÈSE D'IMINES SYMÉTRIQUES

(30) Priority: 04.02.2019 PL 42880819
(43) Date of publication of application: 05.08.2020
(73) Proprietor: Wojskowy Instytut Techniki Inzynieryjnej im. profesora Jozefa Kosackiego, 50-540 Wroclaw (PL)
(72) Inventor: BOGDANOWICZ, Krzysztof Artur, 50-540 Wroclaw (PL); IWAN, Agnieszka, 53-341 Wroclaw (PL)
(74) Representative: Kancelaria Eupatent.pl Sp. z.o.o

(56) References cited:
- WO-A1-2015/142173
- BIN-BIN MA ET AL: "Visualized acid-base discoloration and optoelectronic investigations of azines and azomethines having double 4-[N,N-di(4-methoxyphenyl)amino]phenyl terminals", JOURNAL OF MATERIALS CHEMISTRY C, vol. 3, no. 29, 1 January 2015 (2015-01-01), pages 7748-7755, XP055623532, UK ISSN: 2050-7526, DOI: 10.1039/C5TC00909J
- MISHRA A ET AL: "Synthesis and characterization of spin-coatable tert-amine molecules for hole-transport in organic light-emitting diodes", TETRAHEDRON LETTERS, ELSEVIER, AMSTERDAM, NL, vol. 47, no. 27, 3 July 2006 (2006-07-03), pages 4715-4719, XP025004386, ISSN: 0040-4039, DOI: 10.1016/J.TETLET.2006.04.117 [retrieved on 2006-07-03]
- YASUO SAEGUSA ET AL: "Synthesis and characterization of novel 1,3,4-oxadiazole- or 1,3,4-thiadiazole-containing wholly conjugated polyazomethines", JOURNAL OF POLYMER SCIENCE, PART A: POLYMER CHEMISTRY, vol. 28, no. 13, 1 December 1990 (1990-12-01), pages 3647-3659, XP055623433, US ISSN: 0887-624X, DOI: 10.1002/pola.1990.080281311
- Michiel l. Petrus ET AL: "Design rules for the preparation of low-cost hole transporting materials for perovskite solar cells with moisture barrier properties", Journal of Materials Chemistry A, vol. 5, no. 48, 1 January 2017 (2017-01-01), pages 25200-25210, XP055681413, GB ISSN: 2050-7488, DOI: 10.1039/C7TA06452G

## Description

### TECHNICAL FIELD

The present invention relates to symmetrical imines and a method of synthesizing symmetrical imines. The symmetrical imines can be applied in particular in photovoltaic devices and solar cells.

### BACKGROUND

Compounds containing imine groups exhibit properties that make them applicable in organic photovoltaic cells (also called solar cells). Organic solar cells are an alternative to the most prevalent silicon-based solar cells.

The structure of a solar cell is built via application of thin films made of polymers and block copolymers, such as poly(3-hexylthiophene-2,5-diyl), poly(3,4-ethylenedioxythiophene), doped poly(styrene sulfonate) or poly(3,4-ethylenedioxythiophene) as conductors and semiconductors. Other small-molecule compounds, such as fullerenes or their derivatives, can be added to these layers.

Various publications, including:
- "Synthesis, spectroscopy, and electrochemical investigation of new conjugated polymers containing thiophene and 1,3,4-thiadiazole in the main chain" (by P.S. Patil et al. J Appl Polymer Sci 2012, Vol. 125, 1882-1889)
- "Bent Shaped 1,3,4-Oxadiazole/Thiadiazole heterocyclic rings containing liquid crystals" (by C. Selvarasu et al. J. Chem. Sci. 2015, Vol. 127, No. 10, pp. 1831-1838)
- "SYNTHESIS, CHARACTERIZATION AND EVALUATION OF ANTICANCER ACTIVITY OF SOME NEW SCHIFF BASES OF 1, 3, 4-THIADIAZOLE DERIVATIVES" (by A. Naskar et al. Int J Pharm Pharm Sci 2015, Vol 7, Issue 3, 397-402)
- "Synthesis and Characterization of Novel 1,3,4-Oxadiazole- or 1,3,4-Thiadiazole-Containing Wholly Conjugated Polyazome-thines" (by Y. Saegusa et al. J. Appl. Polym. Sci. Part A: Polym. Chem. 1990, Vol. 28, pp. 3647-3659)
present methods of obtaining asymmetric azomethines or systems containing thiadiazole structures via multi-stage reactions. However, that prior art does not provide examples of the use of these compounds in photovoltaic devices. There are no known examples of symmetrical imines containing thiadiazole groups and the method of obtaining thereof.

### SUMMARY

The present invention relates to symmetrical imines and a method for synthesizing thereof according to the appended claims.

Consequently, the method allows for a single-stage synthesis of symmetric imines, short reaction time and simple purification process, which significantly reduces the production cost, when compared to other imines. The method also allows to obtain symmetric imines that have layer-forming properties. Another advantage of the method is a simplified production process for solar cells due to the symmetric imines being applied directly from the solution, forming a thin film. Typically, solar cells that comprise organic compounds acting as electron donor or acceptor materials are made using various printing methods, i.e. roll-to-roll printing process. For this reason, active organic compounds are processed in form of inks capable of forming continuous coatings. Hence, the new organic compounds need to meet this requirement.

### DETAILED DESCRIPTION

The invention will be presented by means of detailed embodiments.

### First embodiment

0.005 mole of 4-(diphenylamino)benzaldehyde was added to 12 cm³ of dimethylacetamide solution containing 0.002 mole of 3,5-diamino-1,2,4-thiadiazole and 10 mol% of p-Tolueneosulfonic acid in an argon atmosphere. The reaction was carried out at a temperature of 170 °C for 24 hours. Then, the obtained mixture was poured into 100 cm³ of water. The formed product was filtered off and first washed with 30 cm³ of ethanol and then with 5 cm³ of acetone. Next, the product was recrystalized in a cold mixture of acetone and hexane (having a temperature of 4°C), in a volumetric proportion of acetone:hexane of 3:10. Following the recrystallisation, the product was filtered off and dried for 24 hours at a temperature of 60°C.

### Second embodiment

0.005 mole of 4-(ditolylamino)benzaldehyde was added to 12 cm³ dimethylacetamide solution containing 0.002 mole of 3,5-diamino-1,2,4-thiadiazole and 10 mol% of p-Tolueneosulfonic acid in an argon atmosphere. The reaction was carried out at a temperature of 170 °C for 48 hours. Then, the obtained mixture was poured into 100 cm³ of water. The formed product was filtered off and first washed with 30 cm³ of ethanol and then with 5 cm³ of acetone. Next, the product was recrystalized in a cold mixture of acetone and hexane (having a temperature of 4°C), in a volumetric proportion of acetone:hexane of 3:10. Following the recrystallisation, the product was filtered off and dried for 24 hours at the temperature of 60°C.

### Third embodiment

0.005 mole of 4-(diphenylamino)benzaldehyde was added to 12 cm³ of dimethylacetamide solution containing 0.002 mole of 3,5-diamino-1,3,4-thiadiazole and 10 mol% of p-Tolueneosulfonic acid in an argon atmosphere. The reaction was carried out at a temperature of 170 °C for 72 hours. Then, the obtained mixture was poured into 100 cm³ of water. The formed product was filtered off and first washed with 30 cm³ of ethanol and then with 5 cm³ of acetone. Next, the product was recrystalized in a cold mixture of acetone and hexane (having a temperature of 4°C), in a volumetric proportion of acetone:hexane of 3:10. Following the recrystallisation, the product was filtered off and dried for 24 hours at the temperature of 60°C.

### Fourth embodiment

0.005 mole of 4-(ditolylamino)benzaldehyde was added to 12 cm³ of dimethylacetamide solution containing 0.002 mole of 3,5-diamino-1,3,4-thiadiazole and 10 mol% of p-Tolueneosulfonic acid in an argon atmosphere. The reaction was carried out at a temperature of 175 °C for 24 hours. Then, the obtained mixture was poured into 100 cm³ of water. The formed product was filtered off and first washed with 30 cm³ of ethanol and then with 5 cm³ of acetone. Next, the product was recrystalized in a cold mixture of acetone and hexane (having a temperature of 4°C), in a volumetric proportion of acetone:hexane of 3:10. Following the recrystallisation, the product was filtered off and dried for 24 hours at the temperature of 60°C.

Table 1 presents a molecular formula, molecular mass and reaction yield for the obtained symmetric imines with the thiadiazole core having the Formula (1), wherein the side chains (R) contain triphenylamine-derivatives.

**Table 1**

| Core (X) | R | Substituent (Y) | Yield [%] | Molecular formula (molecular mass) |
|---|---|---|---|---|
| 1,2,4-thiadiazole | 4-(diphenylamino)fenylo- | -H | 33 | C₄₀H₃₀N₆S |
| | | | | 626.77 |
| 1,2,4-thiadiazole | 4-(ditolylamino) fenylo- | -CH₃ | 44 | C₄₄H₃₈N₆S |
| | | | | 682.88 |
| 1,3,4-thiadiazole | 4-(diphenylamino)fenylo- | -H | 31 | C₄₀H₃₀N₆S |
| | | | | 626.77 |
| 1,3,4-thiadiazole | 4-(ditolylamino) fenylo- | -CH₃ | 34 | C₄₄H₃₈N₆S |
| | | | | 682.88 |

Table 2 presents examples of ¹H NMR spectra for symmetric imines with thiadiazole core with the Formula (1), wherein the side chains (R) contain triphenylamine-derivatives.

**Table 2**

| Core (X) | R | Substituent (Y) | Signals ¹H NMR [δ, ppm] |
|---|---|---|---|
| 1,2,4-thiadiazole | 4-(diphenylamino) fenylo- | -H | 8.31 (2H, d, -HC=N-); 7.65-7.55 (8H, m, arom. -Ph-); 7.4-7.0 (20 H, arom. -Ph) |
| 1,2,4-thiadiazole | 4-(ditolylamino) phenylo- | -CH₃ | 8.31 (2H, d, -HC=N-); 7.65-755 (8H, m, arom. -Ph-); 7.4-7.0 (16 H, arom. -Ph-); 2.35 (12H, t, -CH₃) |
| 1,3,4-thiadiazole | 4-(diphenylamino) phenylo- | -H | 8.56 (2H, s, -HC=N-); 7.65-7.55 (8H, m, arom. -Ph-); 7.4-7.0 (20 H, arom. -Ph) |
| 1,3,4-thiadiazole | 4-(ditolylamino) phenylo- | -CH₃ | 8.55 (2H, s, -HC=N-); 7.75-7.55 (8H, m, arom. -Ph-); 7.2-6.90 (16 H, arom. -Ph-); 2.35 (12H, s, -CH₃) |

## Claims

1. Symmetrical imines having a molecular structure according to Formula (1): 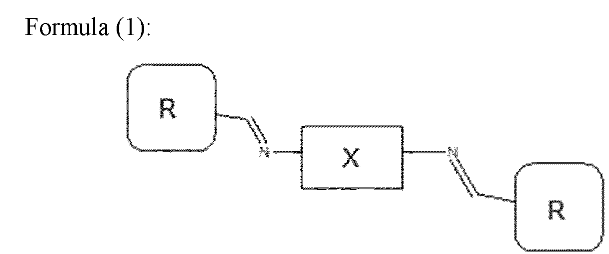 wherein the core (X) is a five-membered thiadiazole ring; and
wherein each side chain (R) have the formula (II): 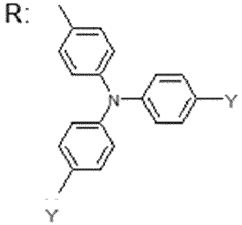 wherein the substituent (Y) is H or CH₃.

2. A method of synthesizing symmetrical imines according to claim 1 the method comprising the steps of:
- in an inert gas atmosphere, adding a five-membered thiadiazole ring derivative to a dimethylacetamide solution comprising derivatives of the side chains (R) of the Formula (II), in a presence of acidic catalyst;
- conducting a reaction in a temperature range from 140°C to 185°C for a period from 24 to 72 hours;
- pouring the obtained mixture to water;
- washing the obtained raw product first with ethanol and afterwards with acetone;
- recrystalising the washed product in a cold mixture of acetone and hexane;
- filtering off and drying the recrystalized product.

3. The method according to claim 2, wherein the five-membered thiadiazole derivative has a form of a diamine.

4. The method according to any of claims 2-3, wherein the derivatives of the side chains (R) of the Formula (II) have a form of an aldehyde.

5. The method according to any of claims 2-4, wherein the inert gas is nitrogen.

6. The method according to any of claims 2-4, wherein the inert gas is argon.

## Patentansprüche

1. Symmetrische Imine, die eine molekulare Struktur gemäß Formel (1) aufweisen: 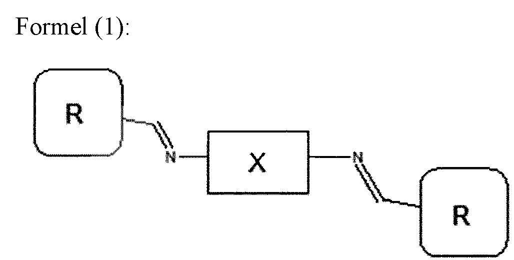 wobei der Kern (X) ein fünfgliedriger Thiadiazolring ist; und
wobei jede Seitenkette (R) die Formel (II) aufweist: 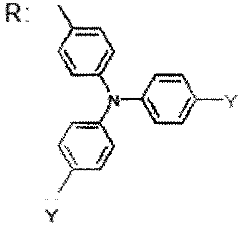 wobei der Substituent (Y) H oder CH₃ ist.

2. Verfahren zur Synthese symmetrischer Imine nach Anspruch 1,
wobei das Verfahren folgende Schritte umfasst:
- in einer Inertgasatmosphäre, Hinzufügen eines fünfgliedrigen Thiadiazolringderivats zu einer Dimethylacetamidlösung, umfassend Derivate der Seitenketten (R) der Formel (II), in Gegenwart eines sauren Katalysators;
- Durchführen einer Reaktion in einem Temperaturbereich von 140 °C bis 185 °C über einen Zeitraum von 24 bis 72 Stunden;
- Gießen des erhaltenen Gemischs in Wasser;
- Waschen des erhaltenen Rohprodukts zuerst mit Ethanol und anschließend mit Aceton;
- Umkristallisieren des gewaschenen Produkts in einem kalten Gemisch aus Aceton und Hexan;
- Abfiltrieren und Trocknen des umkristallisierten Produkts.

3. Verfahren nach Anspruch 2, wobei das fünfgliedrige Thiadiazolderivat eine Form eines Diamins aufweist.

4. Verfahren nach einem der Ansprüche 2-3, wobei die Derivate der Seitenketten (R) der Formel (II) eine Form eines Aldehyds aufweisen.

5. Verfahren nach einem der Ansprüche 2-4, wobei das Inertgas Stickstoff ist.

6. Verfahren nach einem der Ansprüche 2-4, wobei das Inertgas Argon ist.

## Revendications

1. Imines symétriques ayant une structure moléculaire selon la Formule (1) : 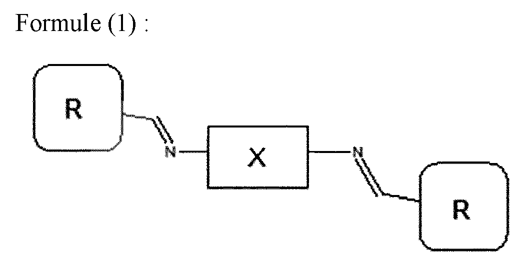 dans lesquelles le cœur (X) est un noyau thiadiazole de cinq éléments ; et
dans lesquelles chaque chaîne latérale (R) a la formule (II) : 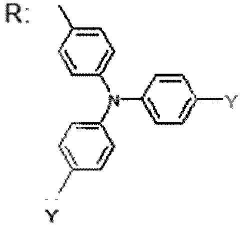 dans lesquelles le substituant (Y) est H ou CH₃.

2. Procédé de synthétisation d'imines symétriques selon la revendication 1, le procédé comprenant les étapes suivantes :
- dans une atmosphère de gaz inerte, ajout d'un dérivé de noyau de thiadiazole de cinq éléments à une solution de diméthylacétamide comprenant des dérivés des chaînes latérales (R) de Formule (II), en présence d'un catalyseur acide ;
- réalisation d'une réaction dans une plage de température allant de 140 °C à 185 °C pendant une période de 24 à 72 heures ;
- versement du mélange obtenu dans de l'eau ;
- lavage du produit brut obtenu d'abord avec de l'éthanol puis avec de l'acétone ;
- recristallisation du produit lavé dans un mélange froid d'acétone et d'hexane ;
- extraction par filtration et séchage du produit recristallisé.

3. Procédé selon la revendication 2, dans lequel le dérivé de thiadiazole de cinq éléments a la forme d'une diamine.

4. Procédé selon l'une quelconque des revendications 2 à 3, dans lequel les dérivés des chaînes latérales (R) de Formule (II) ont la forme d'un aldéhyde.

5. Procédé selon l'une quelconque des revendications 2 à 4, dans lequel le gaz inerte est l'azote.

6. Procédé selon l'une quelconque des revendications 2 à 4, dans lequel le gaz inerte est l'argon.
